# EUROPEAN PATENT APPLICATION

(11) **EP 2 502 585 A1**
(43) Date of publication of application: **26.09.2012**
(21) Application number: 12160681.8
(22) Date of filing: 22.03.2012
(51) Int. Cl.: A61B 17/34, A61B 17/30

(54) **Retention member for laparoscopic access device**

(30) Priority: 14.03.2012 US 201213419759; 23.03.2011 US 201161466553 P
(71) Applicant: Tyco Healthcare Group LP, Mansfield, MA 02048 (US)
(72) Inventor: Rodrigues, Anibal, Milford, CT Connecticut 06460 (US)
(74) Representative: Soames, Candida Jane

(57) **Abstract**

A surgical access port and method of using is disclosed, the surgical access port comprising a cylindrical member defining a longitudinal axis and having proximal ends defined by a pair of rims oriented substantially transverse to the longitudinal axis. At least one lumen extends from the proximal end to the distal end of the cylindrical member, the lumens substantially parallel to the longitudinal axis. A sealing member having proximal and distal ends with opposing openings is disposed externally of the cylindrical member, the opening at the proximal end of the sealing member in contact with the proximal end of the cylindrical member.

## Description

### CROSS REFERENCE TO RELATED APPLICATION

The present application claims the benefit of and priority to U.S. Provisional Application Serial No.61/466,553, filed on March 23, 2011, the entire contents of which are incorporated herein by reference.

### BACKGROUND

### 1. Technical Field

The present disclosure relates to an access port for use in minimally invasive surgical procedures, such as endoscopic or laparoscopic-type procedures, and more particularly to a surgical access port with an external sealing member for body surfaces.

### 2. Background of Related Art

Today, many surgical procedures are performed through small incisions in the skin, as compared to the larger incisions typically required in traditional procedures, in an effort to reduce both trauma to the patient and recovery time. Generally, such procedures are referred to as endoscopic, unless performed on the patient's abdomen, in which case the procedure is referred to as laparoscopic. Throughout the present disclosure, the term minimally invasive should be understood to encompass both endoscopic and laparoscopic procedures. During a typical minimally invasive procedure, surgical objects, such as surgical access ports (e.g., trocar and/or cannula assemblies), endoscopes, or other instruments, are inserted into the patient's body through the incision in tissue. Prior to the introduction of the surgical object into the patient's body, insufflation gases may be used to enlarge the area surrounding the target surgical site to create a larger, more accessible work area. Accordingly, the maintenance of a substantially fluid-tight seal is desirable so as to inhibit the escape of the insufflation gases and the deflation or collapse of the enlarged surgical site.

To this end, various access members are used during the course of minimally invasive procedures and are widely known in the art. A continuing need exists for an access member of a universal size that can be inserted into a variety of tissue incision sites and maintain the conditions of the insufflated surgical site. It is desirable to accommodate a variety of tissue incisions and body surface conditions, and adapt to changing conditions at the surgery site.

### SUMMARY

In accordance with various embodiments, the present disclosure is directed toward a surgical access port having an external sealing member. The surgical access port includes a cylindrical member that defines a longitudinal axis and has proximal and distal ends. The proximal and distal ends of the cylindrical member are defined by a pair of rims or flanges that are oriented substantially transverse to the longitudinal axis. At least one lumen extends through the cylindrical member from the proximal end to the distal end; the lumen is substantially parallel to the longitudinal axis. The surgical access port may be inserted into an incision site such that the rims at the proximal and distal ends of the cylindrical member anchor the surgical access port into a body member.

The surgical access port also includes a sealing member having opposing openings at proximal and distal ends of the sealing member. The opening at the proximal end of the sealing member is in contact with the proximal end of the sealing member, and may engage the rim at the proximal end of the cylindrical member. The sealing member may form a substantially fluid-tight seal with a body surface.

In embodiments, the sealing member may also include a fluid inlet port incorporating a valve to control the flow of fluids into or out of the sealing member. The sealing member may also include a port for the receipt of insufflation fluid. In other configurations, the distal end of the sealing member may be attached to body surface with the aid of an adhesive. Further, the sealing member may be shaped or contoured to contact a contoured body surface or a body surface region with a particular geometry.

Also disclosed is a method of positioning a surgical access port involving placing the surgical access port in a body member and placing the sealing member in contact with a body surface such that a substantially fluid-tight seal is formed. With the surgical access port in place, surgical instruments can be inserted in the lumens and minimally invasive procedures performed in an internal body cavity. When the minimally invasive procedure is completed the surgical instruments and surgical access port can be removed from the body member.

The various aspects of this disclosure will be more readily understood from the following detailed description when read in conjunction with the appended drawings.

### BRIEF DESCRIPTION OF THE DRAWINGS

FIG. 1 is a side perspective view of a surgical access port having a sealing member and disposed in a layer of tissue;

FIG. 2 is a side profile view of the surgical access port of FIG. 1, disposed in a layer of tissue and showing lumens in phantom view extending the length of a cylindrical member;

FIG. 3 is a top plan view of the surgical access port of FIG. 1, showing the sealing member disposed below partially in phantom view;

FIG. 4 is a side profile view of the surgical access port of FIG. 1, with the sealing member engaged by an operator and the formation of a substantially fluid-tight seal between the sealing member and a body surface being shown;

FIG. 5 is a side profile view of the surgical access port of FIG. 1, with surgical instruments inserted through the lumens and into the internal body cavity below;

FIG. 6 is a side profile view of the surgical access port of FIG. 1, with fluid flowing into the space between the sealing member and the body surface;

FIG. 7 is a side profile view of the surgical access port of FIG. 1, disposed in a layer of tissue and having adhesive disposed between a distal end of the sealing member and the body surface; and

FIG. 8 is an embodiment of a surgical access port shown in side profile view, disposed in a layer of tissue and engaging a contoured body surface.

### DETAILED DESCRIPTION OF EMBODIMENTS

The present disclosure will now describe in detail embodiments of a surgical access port with reference to the drawings in which like reference numerals designate identical or substantially similar parts in each view. Throughout the description, the term "proximal" will refer to the portion of the assembly closest to the operator, whereas the term "distal" will refer to the portion of the assembly farthest from the operator. Although discussed in terms of an incision for a minimally invasive procedure, the presently disclosed surgical access port may be used in any naturally occurring orifice (e.g. mouth, anus, or vagina).

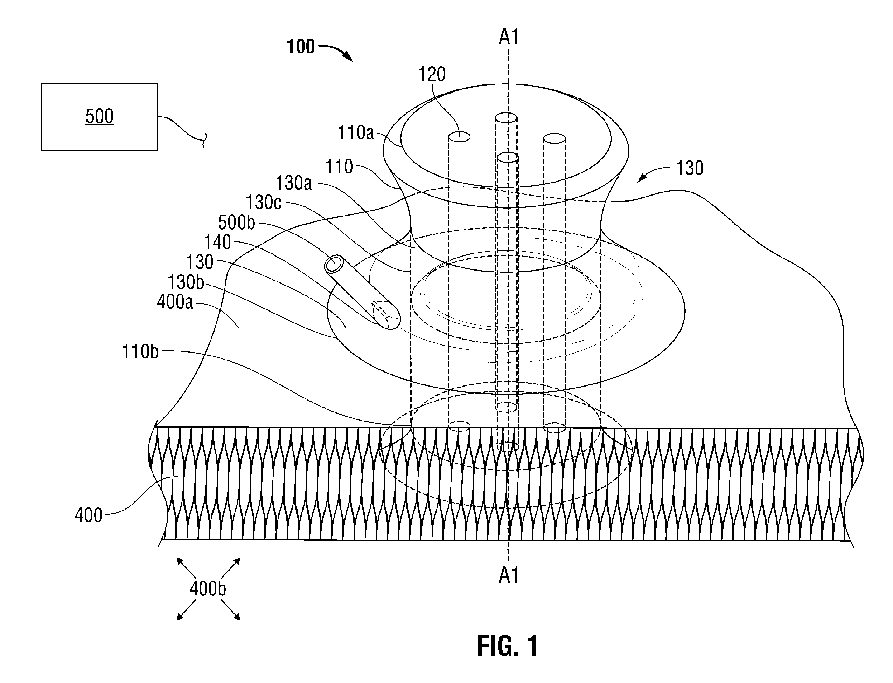

Referring initially to FIG. 1, a surgical access port 100 is shown. The surgical access port 100 includes a cylindrical member 110 having a generally hourglass shape, a proximal end 110a and a distal end 110b, and defines a longitudinal axis A1. The proximal end 110a and the distal end 110b of the cylindrical member 110 are substantially perpendicular to the longitudinal axis A1 and are each defined by a rim. The rims at the proximal end 110a and distal end 110b of the cylindrical member 110 may serve to anchor the surgical access port 100 into a layer of tissue 400.

Extending through the cylindrical member 110 along the longitudinal axis A1 is at least one lumen 120, and in embodiments, multiple lumens 120. The lumens 120 are disposed substantially parallel to the longitudinal axis A1. An access port of the type generally described above is disclosed in U.S. Patent Application Publication Nos. 2009/0093752 A1 and 2010/0240960 A1, the entire disclosures of which are incorporated by reference herein.

Surgical access port 100 also includes a sealing member 130 disposed on the outside of the cylindrical member 110. The sealing member has a proximal end 130a and a distal end 130b, each end having opposing openings defining a passage therethrough for receiving the cylindrical member 110. The sealing member 130 is disposed on an outer surface of the cylindrical member 110 such the proximal end 130 of the sealing member 130 is in contact with the proximal end 110a of the cylindrical member 110. The proximal end 130a of the sealing member 130 may engage the rim at the proximal end 110a of the cylindrical member 110.

Distal end 130b of sealing member 130b may contain a sidewall 130c surrounding the passage for receiving the cylindrical member 110. Sidewall 130c extends from the opening in the proximal end 130a of the sealing member 130 to at least a body surface 400a, and in embodiments, further into the layer of tissue 400. The sidewall 130c serves to partition the space beneath the sealing member 130 from the cylindrical member 110. Further, sidewall 130c sealably engages the outer surface of cylindrical member 110, securing cylindrical member 110 in place and inhibiting the escape of insufflation gases from an internal body cavity 400b.

Sealing member 130 and cylindrical member 110 may be formed as a single unit, or may be separable components. Having the sealing member 130 and the cylindrical member 110 separable from each other allows the cylindrical member 110 to be removed and replaced while utilizing the same sealing member 130.

The body of sealing member 130 is formed of a flexible, yet resilient material suitable to retain fluid and remain in contact with a body member, e.g., a polymeric material. The sealing member 130 is configured and dimensioned to form a substantially fluid-tight seal with a body surface 400a. Such a seal is accomplished when fluids are expelled from the space between sealing member 130 and body surface 400a.

To aid in the evacuation of fluids from the space between the distal end 130b of the sealing member 130 and the sidewall 130c, a fluid release port 140 may be provided. Fluid release port 140 may include a valve suitable for the purpose of controlling the flow of fluids into or out of the sealing member 130, e.g., a duckbill valve or a bi-valve. Fluid release port may further be coupled to a source of vacuum 500 to aid in the evacuation of fluids from sealing member 130. Source of vacuum 500 may be any source capable of drawing fluid from the space between the distal end 130b of the sealing member 130 and the sidewall 130c, e.g., a syringe or pump. Source of vacuum 500 may be coupled to the fluid release port 140 with a tube 500b.

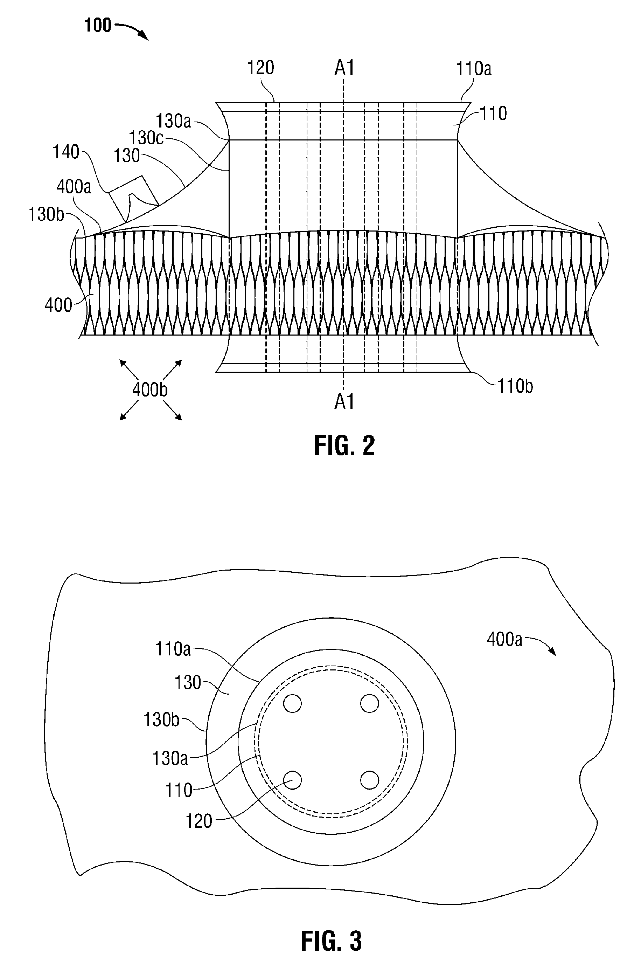

Turning now to FIG. 2, a side profile view of the surgical access port 100 of FIG. 1 is shown. In this view, the placement of the sealing member 130 relative to the components of cylindrical member 110 is shown. The proximal end 130a of the sealing member 130 is shown in contact with the rim at the proximal end 110a of the cylindrical member 110. The distal end 130b of the sealing member 130 is shown covering a body surface 400a. The cylindrical member 110 is shown inserted through a layer of tissue 400, with the distal end 110b of the cylindrical member 110 disposed in an internal body cavity 400b.

Referring to FIG. 3, a top plan view of the surgical access port 100 of FIG. 1 is shown disposed in a layer of tissue 400 (FIG. 1). The relative spacing of the sealing member 130, the cylindrical member 110, and the lumens 120 are shown in this view. The lumens 120 provide an unobstructed path from an area proximal of the cylindrical member 110 to an internal body cavity 400b (FIG. 2).

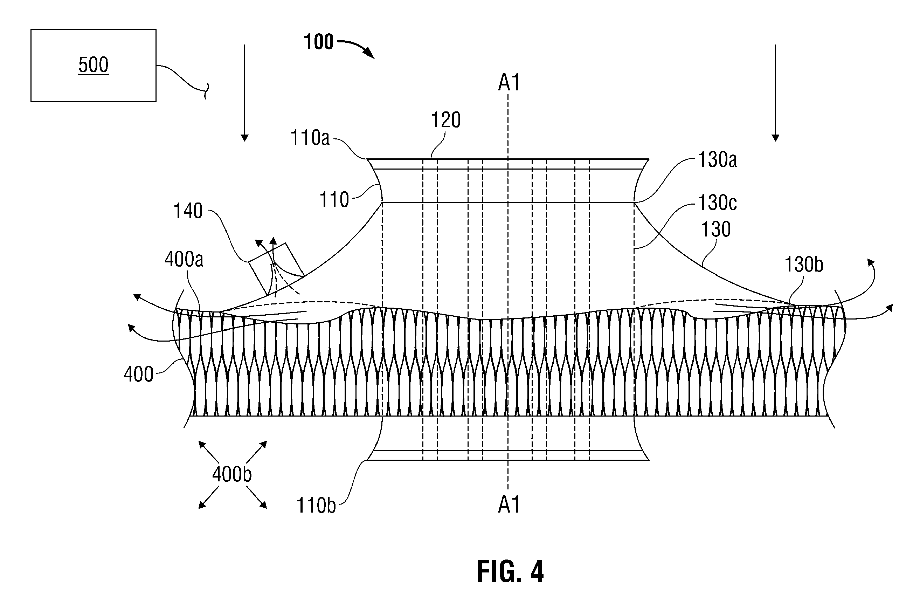

Turning now to FIG. 4, the surgical access port 100 is shown disposed in a layer of tissue 400 and being engaged by an operator. As force is applied in a distal direction along the longitudinal axis, fluid trapped in the space between the distal end 130b of the sealing member 130 and sidewall 130c is shown being forced out the fluid release port 140 and from beneath the distal end 130b of the sealing member 130. Alternatively, fluid release port 130 may be coupled to source of vacuum 500 to aid in the evacuation of fluids from the sealing member as discussed above.

With the evacuation of the fluid in the space between the distal end 130b of the sealing member 130 and the sidewall 130c, a substantially fluid-tight seal is formed. The sealed engagement of sidewall 130c with an outer surface of cylindrical member 110 inhibits insufflation gases from escaping internal body cavity 400b. This is especially important in situations where the cylindrical member 110 is sized smaller than the incision in the tissue layer 400, and insufflation fluids are more likely to escape the internal body cavity 400b. Thus, a surgical access port 100 may have a cylindrical member 110 of universal size, and a sealing member 130 that can be adapted to a variety of surgical sites.

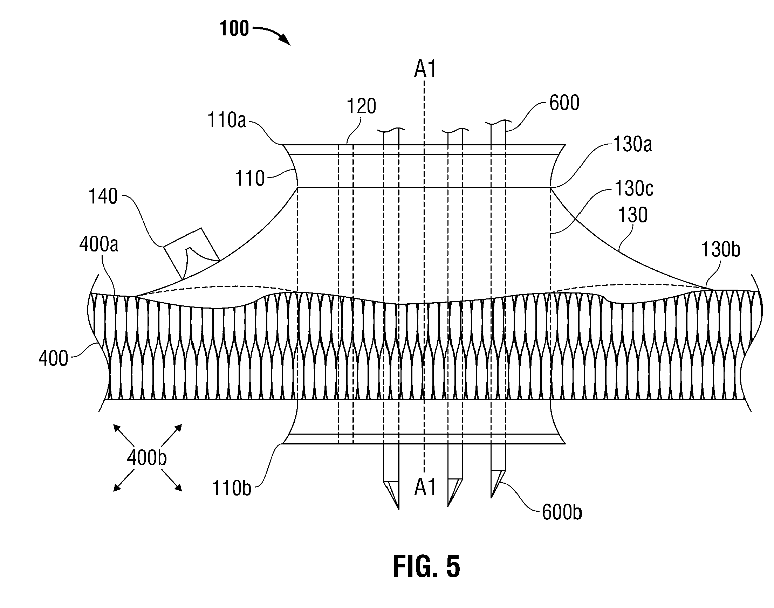

Referring to FIG. 5, the surgical access port 100 is shown disposed in a layer of tissue 400 with surgical instruments 600 inserted through the lumens 120. The surgical instruments 600 and end effectors 600b are thus inserted into an internal body cavity 400b. An operator of the surgical access port 100 can thus perform a minimally invasive procedure in the internal body cavity 400b by manipulating the surgical instruments 600 from an area proximal of the cylindrical member 110 and causing the end effectors 600b to perform desired tasks.

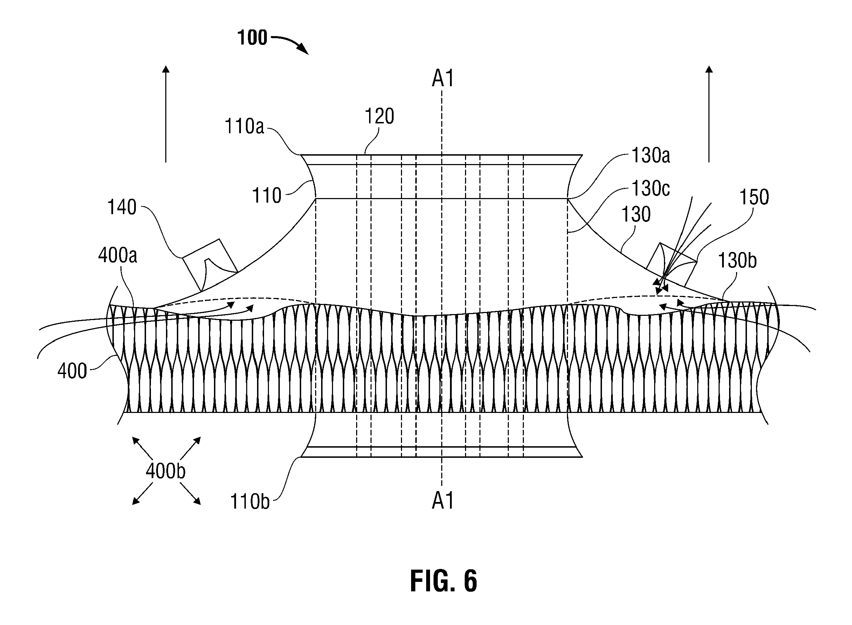

Turning now to FIG. 6, the surgical access port 100 is shown disposed in a layer of tissue with the sealing member 130 being released from a sealed condition with body surface 400a. Also shown is a fluid inlet port 150 disposed on the sealing member. The fluid inlet port 150 functions similarly to fluid outlet port 140 in that it controls the flow of fluids into or out of the sealing member 130. Fluid inlet port 150 may be a valve suitable for such a purpose, e.g., a duckbill valve or a bi-valve. Fluid inlet port 150 may also transmit insufflation gases into the sealing member 130 and any areas in fluid communication therein.

Force may be applied in a direction proximally along the longitudinal axis, as shown, to cause the seal 130 to release from body surface 400a. Alternatively, the fluid inlet port 150 may be manipulated such that fluid flows into the fluid inlet port 150 and sealing member 130.

Fluid is shown flowing into fluid inlet port 150 as well as under distal end 130b of sealing member 130. As fluid fills the space between the distal end 130b of the sealing member 130 and the sidewall 130c, the sealed relation between sealing member 130 and body surface 400a dissipates.

In use, an operator positions the cylindrical member of the surgical access port in place in a layer of tissue 400. Sealing member 130 is engaged by an operator and pressed into a substantially fluid-tight seal with a body surface 400a, as shown in FIG. 4. Alternatively, sealing member 130 may be coupled to a source of inflation fluid 500 to form a substantially fluid-tight seal. Surgical instruments 600 (FIG. 5) are inserted through the lumens 120, with the end effectors 600b (FIG. 5) of the surgical instruments 600 disposed in the internal body cavity 400b. With the surgical instruments 600 in place through the surgical access port 100, minimally invasive procedures may be performed in the internal body cavity 400b. When such procedures are complete, the surgical instruments 600 may be removed from the internal body cavity 400b. The sealed relation between the sealing member 130 and the body surface 400a is dissipated by allowing fluid to flow into the sealing member 130 as shown in FIG. 6, and the surgical access port 100 can then be removed from the layer of tissue 400.

Alternatively, the sealing member 130 may be placed and sealed to body surface prior to the introduction of cylindrical member 110. Cylindrical member 110 may be inserted thereafter. Similarly, in removing the surgical access port 100, the sealed relation of the sealing member 130 and body surface 400a may be maintained due to the presence of sidewall 130c, allowing the cylindrical member 110 to be removed prior to the sealing member 130.

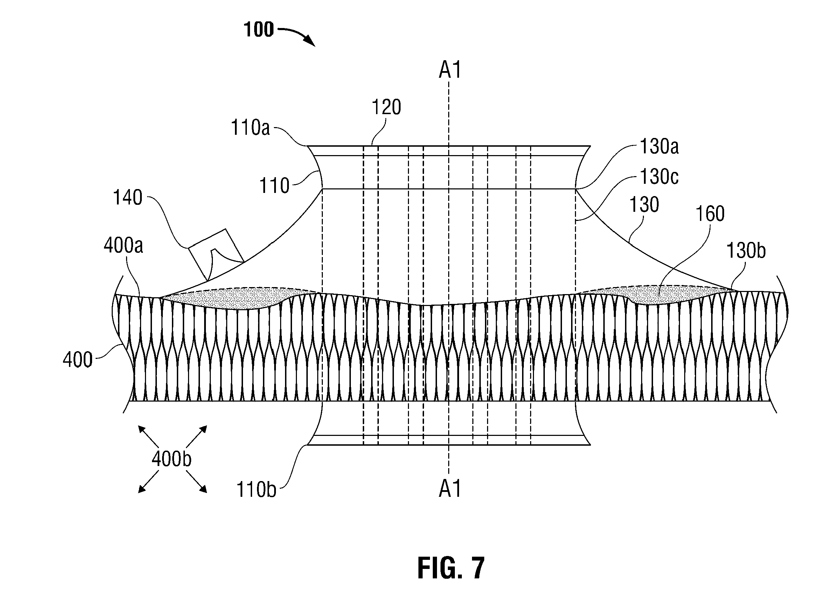

Turning now to FIG. 7, a side profile view of the surgical access port 100 is shown, with adhesive 160 disposed between the distal end 130b of the sealing member 130 and the body surface 400a. Adhesive 160 aids sealing member 130 in maintaining a substantially fluid-tight seal between sealing member 130, cylindrical member 110, and body surface 400a. Adhesive 160 should be a biocompatible adhesive including, but not limited to, adhesives which cure upon tissue contact, which cure upon exposure to ultraviolet (UV) light, which are two-part systems kept isolated from one another and cure upon coming into contact with one another, which are pressure sensitive, which are any combinations thereof, or any other known suitable adhesive.

When fluids are introduced or expelled from the space between the distal end 130b of the sealing member 130 and the sidewall 130c, as shown in FIGS. 4 and 6 above, adhesive 160 may interfere with the flow of fluids beneath distal end 130b of sealing member 130, and as such fluid outlet port 140 and fluid inlet port 150 (FIG. 6) may play a more substantial role in the inlet and expulsion of fluids from the space between the distal end 130b of the sealing member 130 and the sidewall 130c. Source of vacuum fluid 500 may also be utilized in such a situation.

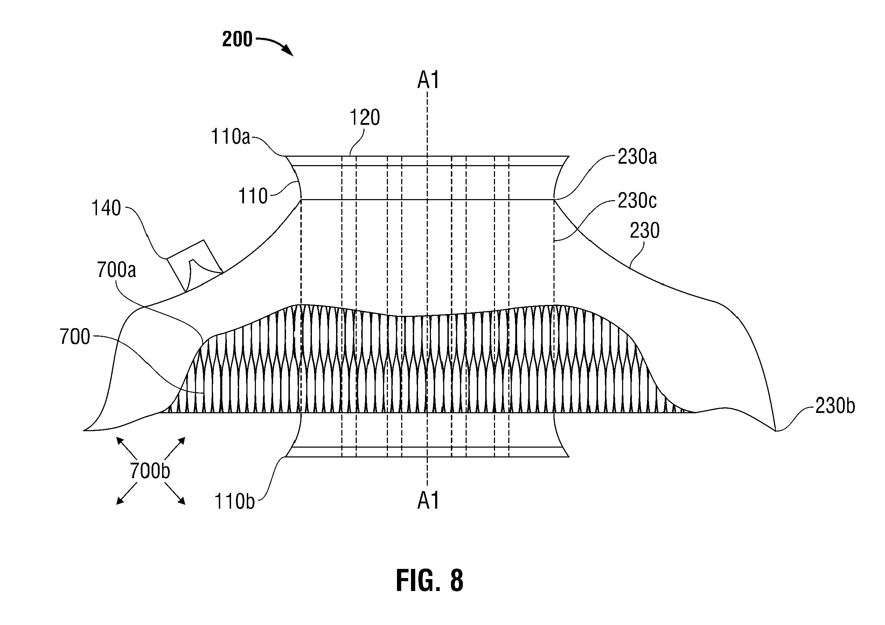

Referring to FIG. 8, an embodiment of a surgical access port 200 is shown disposed in a layer of tissue 700. Layer of tissue 700 has an irregular shape and thus has an irregular body surface 700a. Similar to surgical access port 100 discussed earlier, surgical access port 200 includes a cylindrical member 110 having a generally hourglass shape, a proximal end 110a and a distal end 110b, and defines a longitudinal axis A1. The proximal end 110a and the distal end 110b of the cylindrical member 110 are substantially perpendicular to the longitudinal axis A1 and are each defined by a rim. The rims at the proximal end 110a and distal end 110b of the cylindrical member 110 may serve to anchor the surgical access port 200 into a layer of tissue 700. Extending from the proximal end 110a to the distal end 110b of the cylindrical member 110 is at least one lumen 120 that is oriented substantially parallel to the longitudinal axis A1.

Disposed on the outside of cylindrical member 110 is sealing member 230. Sealing member 230 has a proximal end 230a and a distal end 230b having opposing openings. As in sealing member 130 discussed earlier, sealing member 230 is disposed on the outer surface of cylindrical member 110 such that the proximal end 230a of sealing member 230 engages proximal end 110a of cylindrical member 110. Sealing member 230 may also incorporate a sidewall 230c to partition the space between the distal end 230b of the sealing member 230 and the sidewall 230c from the internal body cavity 400b. Distal end 230b of sealing member 230 is shaped or contoured such that it engages a body surface 400a with a particular surface geometry. Such a surface geometry may be uneven or otherwise irregular because of its particular location on the body, such as at a joint or near a limb. Surgical access port 200 functions in substantially the same manner as described earlier with respect to surgical access port 100, and may include a fluid inlet port 140 to aid in forming a substantially fluid-tight seal between sealing member 130 and body surface 700a.

It will be understood that various modifications may be made to the embodiments disclosed herein. Therefore, the above description should not be construed as limiting, but merely as exemplifications of embodiments. Those skilled in the art will envision other modifications within the scope and spirit of the present disclosure.

The invention may be described by reference to the following numbered paragraphs:-

A surgical access port, comprising:
a cylindrical member defining a longitudinal axis and having proximal and distal ends defined by a pair of rims oriented substantially transverse to the longitudinal axis;
at least one lumen extending from the proximal end to the distal end of the cylindrical member, the lumens substantially parallel to the longitudinal axis; and
a sealing member having proximal and distal ends with opposing openings, the opening at the proximal end of the sealing member in contact with the proximal end of the cylindrical member.

The surgical access port of paragraph [00041], wherein the sealing member further comprises a fluid inlet port.

The surgical access port of paragraph [00041], wherein the sealing member further comprises a fluid release port.

The surgical access port of paragraph [00041], wherein the sealing member has a port for the receipt of insufflation fluid.

The surgical access port of paragraph [00041], wherein the distal end of the sealing member is attached to a body surface with an adhesive.

The surgical access port of paragraph [00041], wherein the sealing member forms a substantially fluid-tight seal with a body surface.

The surgical access port of paragraph [00041], wherein the sealing member is configured and dimensioned to sealably contact a contoured body surface.

The surgical access port of paragraph [00041], wherein the rims are in contact with a body member.

A method of using a surgical access port, comprising the steps of:
placing a surgical access port in a body member, the surgical access port comprising:
   a cylindrical member defining a longitudinal axis and having proximal and distal ends defined by a pair of rims oriented substantially transverse to the longitudinal axis;
   at least one lumen extending from the proximal end to the distal end of the cylindrical member substantially parallel to the longitudinal axis; and
   a sealing member having proximal and distal ends with opposing openings
   the opening at the proximal end of the sealing member in contact with the proximal end of the cylindrical member; and
   placing the sealing member in contact with a body surface such that a substantially fluid-tight seal is formed.

The method of paragraph [00049], further comprising inserting at least one surgical instrument through the at least one lumen.

The method of paragraph [00050], further comprising performing a minimally invasive procedure through the surgical access port.

The method of paragraph [00051], further comprising the step of removing the at least one surgical instrument from the at least one lumen.

The method of paragraph [00052], further comprising the step of removing the surgical access port from the body member.

The method of paragraph [00049], wherein the sealing member further comprises a fluid inlet port.

The method of paragraph [00049], wherein the sealing member further comprises a fluid release port.

The method of paragraph [00049], wherein the sealing member has a port for the receipt of insufflation fluid.

The method of paragraph [00049], wherein the distal end of the sealing member is attached to a body surface with an adhesive.

The method of paragraph [00049], wherein the sealing member forms a substantially fluid-tight seal with a body surface.

The method of paragraph [00049], wherein the sealing member is configured and dimensioned to sealably contact a contoured body surface.

The method of paragraph [00049], wherein the rims are in contact with a body member.

## Claims

1. A surgical access port, comprising:
a cylindrical member defining a longitudinal axis and having proximal and distal ends defined by a pair of rims oriented substantially transverse to the longitudinal axis;
at least one lumen extending from the proximal end to the distal end of the cylindrical member, the lumens substantially parallel to the longitudinal axis; and
a sealing member having proximal and distal ends with opposing openings, the opening at the proximal end of the sealing member in contact with the proximal end of the cylindrical member.

2. The surgical access port of claim 1, wherein the sealing member further comprises a fluid inlet port.

3. The surgical access port of claim 1 or claim 2, wherein the sealing member further comprises a fluid release port.

4. The surgical access port of any preceding claim, wherein the sealing member has a port for the receipt of insufflation fluid.

5. The surgical access port of any preceding claim, wherein the distal end of the sealing member is attached to a body surface with an adhesive.

6. The surgical access port of any preceding claim, wherein the sealing member forms a substantially fluid-tight seal with a body surface.

7. The surgical access port of any preceding claim, wherein the sealing member is configured and dimensioned to sealably contact a contoured body surface.

8. The surgical access port of any preceding claim, wherein the rims are in contact with a body member.
